# EUROPEAN PATENT SPECIFICATION

(11) **EP 3 609 394 B1**
(45) Date of publication and mention of the grant of the patent: **16.04.2025**
(21) Application number: 18785180.3
(22) Date of filing: 07.02.2018
(51) Int. Cl.: A61B 5/021, A61B 5/0225, A61B 5/0235

(54) **BLOOD PRESSURE MEASUREMENT DEVICE WEARABLE BY A PATIENT**
VON EINEN PATIENTEN AM KÖRPER TRAGBARE BLUTDRUCKMESSVORRICHTUNG
DISPOSITIF DE MESURE DE LA TENSION ARTÉRIELLE POUVANT ÊTRE PORTÉ PAR UN PATIENT

(30) Priority: 11.04.2017 US 201762484092 P; 24.01.2018 US 201815878686
(43) Date of publication of application: 19.02.2020
(73) Proprietor: Becton, Dickinson and Company, Franklin Lakes, NJ 07417 (US)
(72) Inventor: AXELROD, Blake, W., Irvine CA 92614 (US); SIEMONS, Alexander, H., Irvine CA 92614 (US)
(74) Representative: Eisenführ Speiser
(86) International application number: PCT/US2018/017203
(87) International publication number: WO 2018/190942

(56) References cited:
- WO-A1-2016/146356
- WO-A2-2007/028107
- CN-U- 204 765 590
- US-A1- 2004 158 162
- US-A1- 2011 105 917
- US-A1- 2012 283 583
- US-A1- 2015 305 632
- MARKANDMEDIA.NL: "Finapres Medical Systems | Products - Finapres Nano Core - OEM module", 28 March 2017 (2017-03-28), XP055673444, Retrieved from the Internet <URL:https://web.archive.org/web/20170328165635/http://www.finapres.com/pagina/182/Finapres?_Nano_Core_-_OEM_module/> [retrieved on 20200304]
- ANONYMOUS: "Meer én minder functionaliteit voor bloeddrukmeter Finapres", 17 March 2015 (2015-03-17), XP055673123, Retrieved from the Internet <URL:https://www.demcon.nl/publicatie-mechatronica-en-machinebouw/> [retrieved on 20200303]
- ADINSTRUMENTS: "Human NIBP Controller Owner's Guide Human NIBP Owner's Guide", 2014, XP055673095, Retrieved from the Internet <URL:http://cdn.adinstruments.com/adi-web/manuals/human-nibp-OG.pdf> [retrieved on 20200303]

## Description

### BACKGROUND

### Field

Embodiments of the invention relate to a blood pressure measurement device that utilizes volume clamping and that is wearable by a patient.

### Relevant Background

Volume clamping is a technique for non-invasively measuring blood pressure in which pressure is applied to a subject's finger in such a manner that venous flow is fully obstructed and arterial pressure may be balanced by a time varying pressure to maintain a constant arterial volume. In a properly fitted and calibrated system, the applied time varying pressure is equal to the arterial blood pressure in the finger. The applied time varying pressure may be measured to provide a reading of the patient's arterial blood pressure.

This may be accomplished by a finger cuff that is arranged around a finger of a patient. The finger cuff may include an infrared light source, an infrared sensor, and an inflatable bladder. The infrared light may be sent through the finger in which a finger artery is present. The infrared sensor picks up the infrared light and the amount of infrared light registered by the sensor may be inversely proportional to the artery diameter and indicative of the pressure in the artery.

In the finger cuff implementation, by inflating the bladder in the finger cuff, a pressure is exerted on the finger artery. If the pressure is high enough, it will compress the artery and the amount of light registered by the sensor will increase. The amount of pressure necessary in the inflatable bladder to compress the artery is dependent on the blood pressure. By controlling the pressure of the inflatable bladder such that the diameter of the finger artery is kept constant, the blood pressure may be monitored in very precise detail as the pressure in the inflatable bladder is directly linked to the blood pressure.

In a typical present day finger cuff implementation, a volume clamp system is used with the finger cuff. The volume clamp system typically includes a pressure generating system and a regulating system that includes: a pump, a valve, and a pressure sensor in a closed loop feedback system that are used in conjunction with the measurement of the arterial volume. To accurately measure blood pressure, the feedback loop provides sufficient pressure generating and releasing capabilities to match the pressure oscillations of the subject's blood pressure.

Unfortunately, present finger cuff systems rely upon very large devices, such as, rotary pumps, blower pumps, and related devices, for the pressure generating system, to generate the pneumatic pressure for the volume clamp system. Such pump devices are comparatively much larger than the finger cuff itself and are located at distant locations to provide pressure to the finger cuff. As such, these types of pumps consume significant power and are physically removed from the patient thereby creating long air paths that hinder performance and that may result in inaccurate blood pressure measurements. Further, the large pump systems and supporting hardware expand clutter and hinder mobility in the hospital, emergency room, intensive care unit (ICU), doctor's office, ambulance, and other patient care locations. Moreover, this type of large pump system requires high energy consumption as a result of the continuously operating pump and provides further disadvantages as to noise due to the pump and the regular discharge of air. These attributes of high energy use, large components, and noise are undesirable in particular health care environments, such as ambulatory use, emergency rooms, intensive care unit (ICU), examination rooms, and in hospital rooms in which measurements are performed while a patient is sleeping.

The Finapres Nano-core OEM module, developed by Finapres Medical Systems, is a non-invasive continuous blood pressure OEM module for integration into blood pressure measurement systems. The Finapres Nano-core OEM module is miniaturized so that it can be mounted to a patient's wrist. It comprises two manifolds for connecting the device with two finger cuffs, sensors and valves, inter alia for switching measurement between two fingers. In order to achieve adequate pressure dynamics with such a miniature system, the Finapres Nano-core OEM module further comprises a system consisting of a plunger and a micro pump. The Nano-core OEM module is intended to be as quiet as possible, therefore the micro pump is switched on as little as possible, only if there is a large change in the average pressure, for example if the patient is quickly moved from lying to sitting.

WO2016/146356A1 provides an apparatus for measuring the blood pressure, BP, of a user, the apparatus comprising a volume-clamp BP monitoring device that comprises a first pressure device for applying pressure to a first part of the body of the user, a first photoplethysmogram, PPG, sensor for obtaining a first PPG signal from the first part of the body of the user, and a control unit that is configured to analyse the first PPG signal and to control the pressure of the first pressure device; wherein the control unit is configured to adjust the pressure of the first pressure device to maintain the first PPG signal at a constant level and to determine the BP of the user from the pressure of the first pressure device; and a second sensor, separate from the first PPG sensor, for measuring a physiological characteristic of the user in a second part of the body of the user, wherein the second part of the body is separate from the first part of the body; wherein the apparatus is configured to analyse the measured physiological characteristic to determine a measure of the blood perfusion in the second part of the body of the user, and to determine whether to perform a recalibration of the volume-clamp BP monitoring device on the basis of changes in the blood perfusion.

US2011/105917A1 provides a system and method of digital control for a blood pressure measurement system. A photo-plethysmographic (PPG) system produces a frequency signal that corresponds to the measured light in the PPG system. Such light may be indicative of blood volume in a vein or artery. The frequency signal may be used to control one or more pressure valves of the system in order to measure blood pressure and hold the frequency signal constant.

WO2007/028107A2 (WO'107) provides a sphygmomanometer cuff assembly, air pump, pressure sensor and release valve are contained in an otherwise conventional computer mouse controller or are attached to a cell phone, television remote control or directly to a computer. In one example the sphygmomanometer cuff is nominally positioned within a mouse structure and is extended outside the mouse housing during the measurement. In another example, the cuff is always external of the mouse structure and is easily connected to the mouse at special ports during the measurement. In yet another example, the cuff is always internal of the mouse structure and is readily accessible through an aperture in the housing surface of the mouse to permit the measurement to take place. In yet another example, a wrist cuff and associated pump, sensor and valve are designed to be selectively connected to a cell phone to which appropriate software has been downloaded from a computer. In yet another example, the cuff and associated components are connected directly to a television remote control unit. In all of the examples shown in WO' 107, the sphygmomanometer cuff is configured for receiving a human finger or wrist in circumambient pressured engagement using controlled air pressure to vary the cuff engagement pressure in a precise manner.

### SUMMARY

The invention relates to blood pressure measurement devices as defined in independent claim 1 that utilizes volume clamping and that are wearable by a patient and to a method for blood pressure measurement as defined in independent claim 7. The blood pressure measurement device may comprise: a finger cuff attachable to the patient's finger including a light emitting diode (LED) - photodiode (PD) pair to measure a pleth signal and a bladder surrounding the finger and a blood pressure measurement controller coupled to the bladder through a finger cuff connector to provide pneumatic pressure to the bladder. The blood pressure measurement controller may be wearable by the patient. The blood pressure measurement controller may include: a pump coupled to the finger cuff connector, a valve, a pressure sensor, and control circuitry coupled to the pump, the valve, the pressure sensor, and the LED-PD pair. The control circuitry may be configured to: control the pneumatic pressure applied by the pump through the finger cuff connector to the bladder to replicate the patient's blood pressure based upon measuring the pleth signal; control the opening of the valve to release pneumatic pressure from the bladder; and measure the patient's blood pressure by monitoring the pressure of the bladder with the pressure sensor.

### BRIEF DESCRIPTION OF THE DRAWINGS

FIG. 1A is a diagram of an example of a blood pressure measurement device, according to one embodiment of the invention.
FIG. 1B is a diagram illustrating components of a finger cuff and a blood pressure management controller, according to one embodiment of the invention.
FIG. 1C is a diagram illustrating components of a finger cuff and a blood pressure management controller, with a fixed orifice, according to one example of the disclosure.
FIG. 2 is a diagram of a chart illustrating the principals of operation of the finger cuff and the blood pressure measurement controller in view of a pleth signal and pressure for implementing a volume clamp mode, according to one embodiment of the invention.
FIG. 3A is a diagram of a chart illustrating pressure, pump commands, and valve commands for implementing a volume clamp mode, according to one embodiment of the invention.
FIG. 3B is a diagram of a chart illustrating pressure and pump commands, in the fixed orifice implementation, for implementing a volume clamp mode, according to one example of the disclosure.
FIG. 4A is a perspective view of the blood pressure measurement controller including a first type of connector to the finger cuff, according to one embodiment of the invention.
FIG. 4B illustrates the internal components of the blood pressure measurement controller of FIG. 4A, according to one embodiment of the invention.
FIG. 5A is a perspective view of the blood pressure measurement controller including a connection fixture to the finger cuff, according to one embodiment of the invention.
FIG. 5B illustrates the internal components of the blood pressure measurement controller of FIG. 5A, according to one embodiment of the invention.

### DETAILED DESCRIPTION

Embodiments of the invention may relate to a pressure regulating feedback loop for volume clamp blood pressure measurement for use with a finger cuff. The feedback loop may contain a pump for generating pressure within a bladder of the finger cuff to compress a subject's artery and a valve for releasing pressure from the bladder. The feedback loop may further contain a pressure sensor that measures pressure within the bladder of the finger cuff and a system for measuring the volume of the clamped artery, such as, by utilizing an infrared light emitting diode (LED) and photodiode (PD).

In particular, embodiments of the invention may relate to a blood pressure measurement device that utilizes volume clamping and that is wearable by a patient. The blood pressure measurement device may comprise: a finger cuff attachable to the patient's finger including a light emitting diode (LED) - photodiode (PD) pair to measure a pleth signal and a bladder surrounding the finger and a blood pressure measurement controller coupled to the bladder through a finger cuff connector to provide pneumatic pressure to the bladder. The blood pressure measurement controller may also be wearable by the patient. The blood pressure measurement controller may include: a pump coupled to the finger cuff connector, a valve, a pressure sensor, and control circuitry coupled to the pump, the valve, the pressure sensor, and the LED-PD pair. As will be described, the control circuitry may be configured to: control the pneumatic pressure applied by the pump through the finger cuff connector to the bladder to replicate the patient's blood pressure based upon measuring the pleth signal; control the opening of the valve to release pneumatic pressure from the bladder; and measure the patient's blood pressure by monitoring the pressure of the bladder with the pressure sensor.

As will be described, according to embodiments of the invention, the finger cuff may be attached to a patient's finger and a blood pressure measurement controller including a pump, a valve, and a sensor may be coupled to the patient's finger, hand, wrist, arm, or other part of the patient's body, or may not be on the patient's body but may be in close proximity to the finger cuff, for a compact and energy efficient system to monitor a patient's blood pressure.

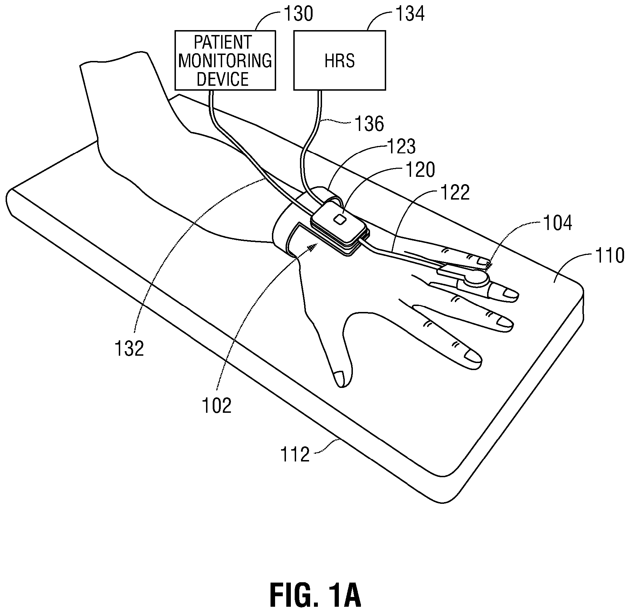

With reference to FIG. 1A, an example of a blood pressure measurement device 102 will be described. As shown in FIG. 1A, the blood pressure measurement device 102 may include a finger cuff 104 having a suitable housing and a suitable finger connector (e.g., including a bladder) that may be attached to a patient's finger and a blood pressure measurement controller 120 that may be attached to the patient's body (e.g., a patient's hand). The blood pressure measurement device 102 may further be connected to a patient monitoring device 130 and a heart reference sensor (HRS) 134. The operations of the blood pressure measurement device 102 including the finger cuff 104 and the blood pressure measurement controller 120 will be hereafter described in more detail.

Continuing with this example, as shown in FIG.1A, a patient's hand may be placed on the face 110 of an arm rest 112 for measuring a patient's blood pressure with the blood pressure measurement device 102. As will be described, the blood pressure measurement controller 120 of the blood pressure measurement device 102 may be coupled to a bladder of the finger cuff 104 through a finger cuff connector 122 in order to provide pneumatic pressure to the bladder for use in blood pressure measurement. Blood pressure measurement controller 120 may be coupled to the patient monitoring device 130 through a power/data cable 132 and to the HRS 134 through a HRS connector 136. The patient monitoring device 130 may be any type of medical electronic device that may read, collect, process, display, etc., physiological readings/data of a patient including blood pressure, as well as any other suitable physiological patient readings. Accordingly, power/data cable may transmit data to and from patient monitoring device 130 and also may provide power from the patient monitoring device 130 to the blood pressure measurement controller 120 and finger cuff 104. The HRS 134 may be placed near the patient's heart level and connected by the HRS connector 136 to the blood pressure measurement controller 120 of the blood pressure measurement device 102 to allow for the compensation of potential errors due to differences in height between the finger cuff 104 and the heart level in the calculation of blood pressure measurements.

As can be seen in FIG.1A, in one example, the finger cuff 104 may be attached to a patient's finger and the blood pressure measurement controller 120 may be attached on the patient's hand with an attachment bracelet 123 that wraps around the patient's wrist. However, it should be appreciated that due to the small size of the blood pressure measurement controller 120 that a wide variety of attachment configurations may be utilized. For example, the blood pressure measurement controller 120 may be placed on a patient's finger (e.g., the same finger as the finger cuff 104 or on one or more different fingers), hand, wrist, arm, or other places such that it is mounted or placed locally to the finger cuff 104 in a convenient fashion. As one particular example, the blood pressure measurement controller 120 may be clipped to a pair of the patient other fingers (e.g., utilizing the attachment bracelet or velcro-strip).

Alternatively, the blood pressure measurement controller 120 may be placed not on the patient's body but may be placed or mounted in close proximity to the finger cuff 104. For example, the blood pressure measurement controller 120 may be clamped or attached to the arm rest 112 (e.g., placed on a clip or be velcroed) near the finger cuff 104 or may simply dangle off of the finger cuff 104 and may not be attached to anything. By having the blood pressure measurement controller 120 removed from the patient's body, access to a patient's arteries and veins is freed-up. Additionally, it should be appreciated that the approximately rectangular formation of the blood pressure measurement controller 120 shown in Figure 1A is merely a design implementation and that any suitable shape may be used. It should further be appreciated that due to the small size of the blood pressure measurement controller 120 that a wide variety of attachment configurations may be utilized, and these are merely examples.

As will be described in more detail hereafter, finger cuff 104 in conjunction with blood pressure measurement controller 120 that includes: a pump, a valve, a pressure sensor and control circuity; may be utilized to measure the patient's blood pressure by monitoring the pressure of the bladder with the pressure sensor and may display the patient's blood pressure on the patient monitoring device 130. In particular, due to the small size of the finger cuff 104 and blood pressure measurement controller 120, techniques are provided to monitor a patient's blood pressure with a compact, mobile, energy efficient, and low noise system.

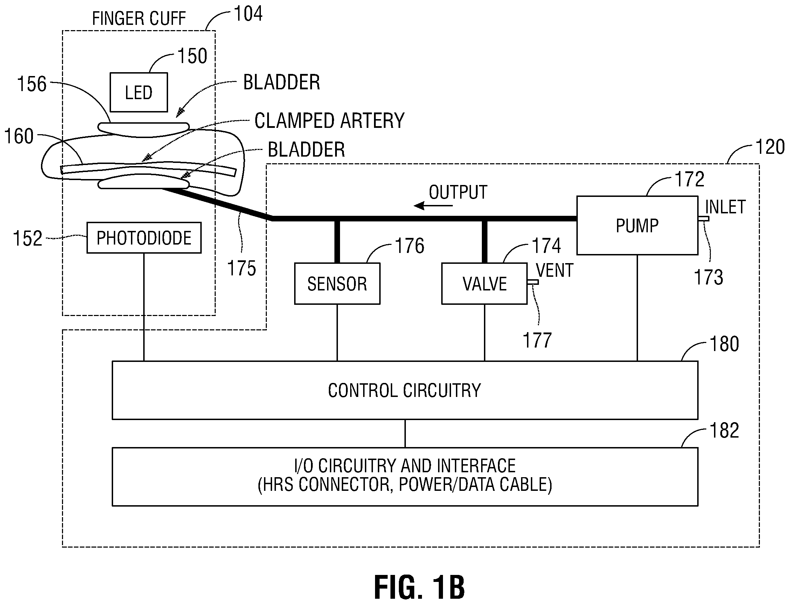

With additional reference to FIG.1B, FIG. 1B is a diagram of an example of a blood pressure measurement device, according to one embodiment of the invention.

Finger cuff 104 may include a light emitting diode (LED) 150 and a photodiode (PD) 152 to create an LED-PD pair. The finger cuff 104 including the LED-PD pair in cooperation with a bladder 156 that surrounds the finger and compresses or clamps against the patient's artery 160 may be used to measure a pleth signal to measure a patient's blood pressure, as will be described.

The blood pressure measurement controller 120 may be coupled to the bladder 156 of the finger cuff 104 through a finger cuff connector 175 to provide pneumatic pressure to the bladder 156. The blood pressure measurement controller 120 may include a pump 172, a valve 174, and a sensor 176. Further, blood pressure measurement controller 120 may include control circuity 180 and input/output circuitry and interfaces 182, as will be described in more detail hereafter. As examples of interfaces, an interface to HRS connector 136 to HRS 134 and an interface to power/data cable 132 to patient monitoring device 130 may be utilized.

As has been described, the blood pressure measurement controller 120 may be wearable by a patient and may be mounted on patient's finger, hand, wrist, arm, or other part of the patient's body or may not be worn by the patient but may just be located locally to the finger cuff 104 (e.g., be placed on the arm rest, be dangling from the patient's finger, etc.). Further, as has been described, the blood pressure measurement controller 120 may be coupled to the finger cuff 104 by a finger cuff connector 175 which may be an appropriate connector 122 that includes a tube portion to provide pneumatic pressure from the pump 172 of the blood pressure measurement controller 120 to the bladder 156 of the finger cuff 104 (as well as a cable portion to provide the LED drive to the finger cuff and the pleth signal from the finger cuff 104 to the control circuity 180) or by a connection fixture for directly connecting pneumatic pressure from the pump 172 of blood pressure measurement controller 120 to the bladder 156 of the finger cuff 104 (as well as to provide the pleth signal from the finger cuff 104 to the control circuity 180) such that the blood pressure measurement controller 120 is mountable on top of the finger cuff 104. Thus, blood pressure measurement controller 120 may be connected locally by a tube or may be directly connected on top of the finger cuff 104 via a connection fixture upon a user's finger. These examples will be described in more detail hereafter. Also, it should be appreciated that the finger cuff connector 175 electronically connects the output of the LED-PD pair to the control circuity 180.

Therefore, in one embodiment, blood pressure measurement controller 120 may include: a pump 172 including an air inlet 173 that is coupled to the finger cuff connector 175 to provide pneumatic pressure to the bladder 156; a valve 174 with a vent 177 coupled to the pneumatic pressure flow to release pneumatic pressure from the bladder 156; a pressure sensor 176 coupled to the pneumatic pressure flow to monitor the pneumatic pressure of the bladder 156; and control circuitry 180 coupled to the pump 172, valve 174, and sensor 176. It should be appreciated that the components of the blood pressure measurement controller 120 may be suitably contained and interconnected within the housing of the blood pressure measurement controller 120 shown in FIG. 1A, but that any suitable housing may be utilized.

The control circuity 180 may be coupled to the pump 172, the valve 174, the sensor 176, and the LED-PD pair in order to control the bladder 156 of the finger cuff 104 and to measure the pleth signal from the LED-PD pair such that the control circuitry 180 of the blood pressure measurement controller 120 can measure a patient's blood pressure.

In particular, control circuitry 180 may be configured to: control the pneumatic pressure applied by the pump 172 (e.g., from air inlet 173) through the finger cuff connector 175 to the bladder 156 (e.g., denoted as output) to replicate the patient's blood pressure based upon measuring the pleth signal received from the LED-PD pair of the finger cuff 104. Further, control circuitry 180 may be configured to: control the opening of the valve 174 via vent 177 to release pneumatic pressure from the bladder 156. Additionally, control circuitry 180 may be configured to: measure the patient's blood pressure by monitoring the pressure of the bladder 156 based upon the input from the pressure senor 176, which should be the same as patient's blood pressure.

The patient's measured blood pressure may be commanded by control circuitry 180 to be transmitted through the I/O circuitry and interface 182 through data/power cable 132 to patient monitoring device 130 to display the patient's measured blood pressure. Further, control circuitry 180 based upon input from HRS 134 and other sources received through the I/O circuitry and interface 182 through HRS connector 136 may allow for compensation of potential errors due to differences in height between the finger cuff 104 and the heart level in the calculation of blood pressure measurements.

It should be appreciated that that control circuitry 180 of blood pressure measurement controller 120 may implement a computational algorithm to control the pneumatic pressure applied by the pump 172 (e.g., output) through the finger cuff connector 175 to the bladder 156 to replicate the patient's blood pressure based upon the measured pleth signal received from the LED-PD pair of the finger cuff 104 in conjunction with controlling the opening of the valve 174 to release pneumatic pressure from the bladder 156 and measuring the patient's blood pressure by monitoring the pressure of the bladder 156 with the pressure sensor 176 and at the same time commanding the calculated blood pressure measurement to be displayed by the patient monitoring device 130. Further, it should be appreciated that the pneumatic pressure applied via the pump 172 may be a suitable gas, such as, air, or in other implementation may utilize a suitable liquid.

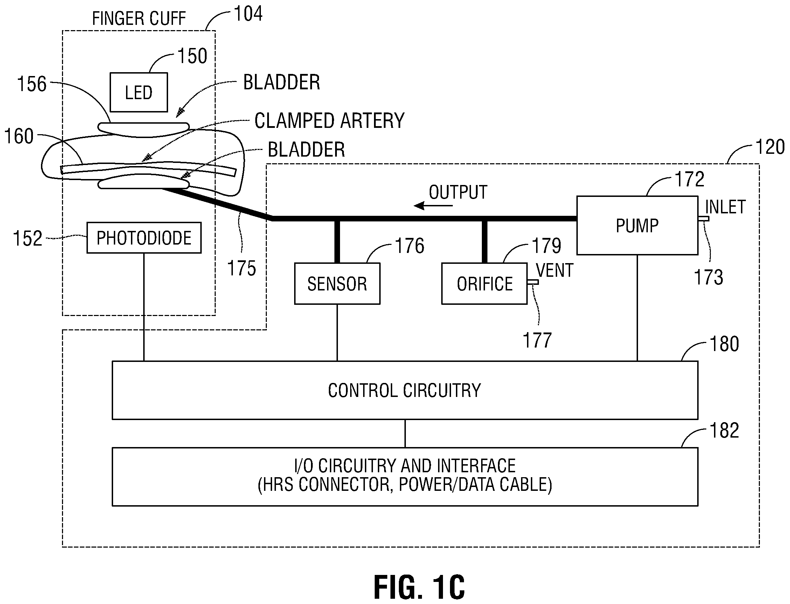

With additional reference to FIG.1C, FIG. 1C is a diagram of an example of a blood pressure measurement device, according to one embodiment of the invention, in which, instead of a valve that is controlled by control circuitry, as previously described, a fixed orifice 179 is utilized. In this example of the disclosure, the fixed orifice 179 including a vent 177 is used to passively release pneumatic pressure from the bladder 156 instead of the valve. Accordingly, the opening and closing of the valve is not required and control of the valve to open and close by the control circuitry 180 is not required, as the fixed orifice 179 passively releases pneumatic pressure from the bladder 156. In this implementation, pneumatic pressure applied to the bladder 156 will be completely controlled and modulated by the pump 172, as controlled by the control circuitry 180, while using a fixed orifice 179, that is not controlled. Examples of this fixed orifice 179 implementation, will be hereafter described in more detail. The rest of the components of FIG. 1C and their functionality are similar to the previous description of FIG. 1B, and will not be repeated for brevity's sake.

Examples of the types of operations implemented by the finger cuff 104 and blood pressure measurement controller 120 will be hereafter described.

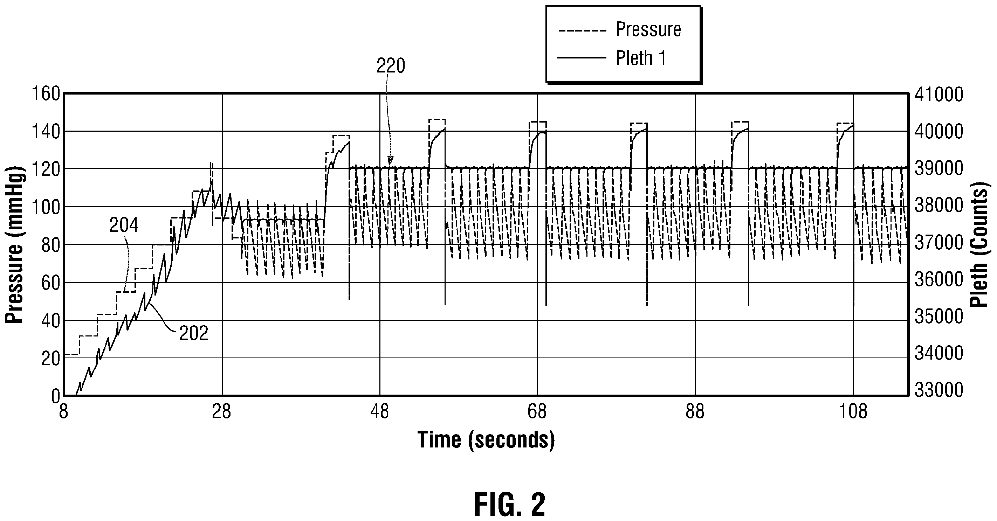

With additional reference to FIG. 2, a brief description of the principals of operation of the finger cuff 104 and the blood pressure measurement controller 120 and the volume clamp mode will be described. In FIG. 2, the X-axis shows time (seconds) and the Y-axis shows pressure (mmHg) and pleth counts. Line 202 shows the pleth signal from the LED-PD pair of the finger cuff 104 received by the control circuity 180 as the pressure shown by line 204 in the bladder 156 is changed by increasing/decreasing pneumatic pressure by pump 172 (e.g., as measured by pressure sensor 176). The pleth signal 202 is inversely proportional to artery volume. As can be seen on FIG. 2, pressure 204 may be increased to the bladder 156 of the finger cuff 104 step-wise by the pump 172 (as measured by pressure sensor 176) and the pleth signal 202 increases accordingly - as the artery is squeezed. The blood pressure beats are visible in the pleth signal during this rise.

In particular, as can be seen in FIG. 2, control circuitry 180 of blood pressure measurement controller 120 chooses a desirable pleth signal 202 received from LED-PD pair and switches to a volume clamp mode, in which, the bladder pressure is controlled by pneumatic pressure applied by pump 172 to bladder 156 (as controlled by control circuitry 180) to keep the pleth signal 202 constant by balancing the blood pressure in the artery 160. For example, as can be seen by section 220, the pleth signal 202 is constant while the pressure signal 204 is constantly changed. In particular, pump 172 is commanded by control circuitry 180 to variably apply pneumatic pressure through the finger cuff connector 175 to the bladder 156 of the finger cuff 104 and valve 174 through vent 177 is variably commanded to open to release pneumatic pressure from the bladder 156, such that, in volume clamp mode, the pressure signal 204 applied to the bladder, as shown in section 220, is equal to the patient's blood pressure (e.g., approximately 120/80).

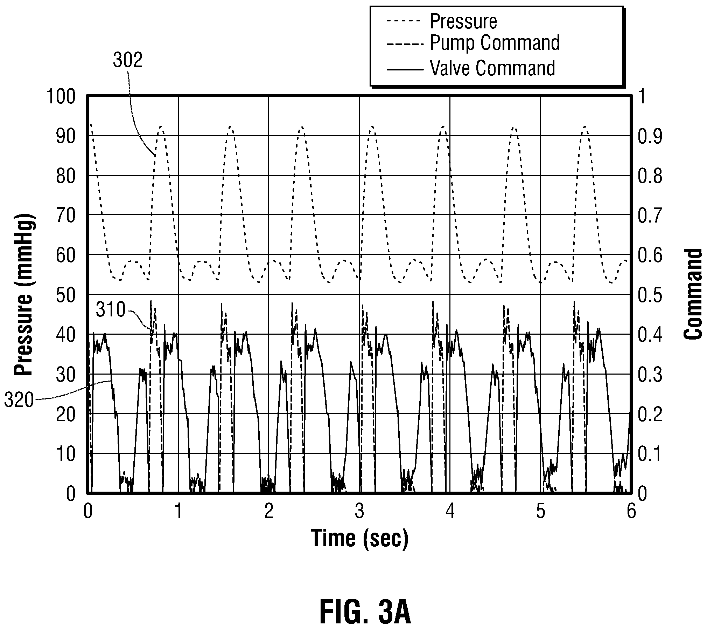

With additional reference to FIG. 3A, an example of the volume clamp mode will be described. In this example, the opening and closing of valve 174 is controlled by control circuitry 180. In FIG. 3A, the X-axis shows time (seconds) and the Y-axis shows pressure (mmHg) and command values (e.g., 0-1). For example, line 302 shows pressure generated in the bladder 156 of the finger cuff 104 by the pump 172 applying pneumatic pressure under the control of control circuitry 180, as a function of time. Below the bladder pressure shown by line 302, line 310 shows a pump drive signal commanding when pump 172 is turned on and how hard it is being driven (e.g., 0-1 command value), as commanded by control circuitry 180. It should be noted that pump 172 is only turned on when the pressure of the bladder 152 is rising. Along with the pump drive signal 310, a valve command signal 320 commanded by control circuitry 180 shows when valve 174 is commanded to be opened and how far it is commanded to be opened (e.g., 0-1 command value). It should be noted that valve 174 is only opened when pressure is dropping, as shown in FIG. 3A.

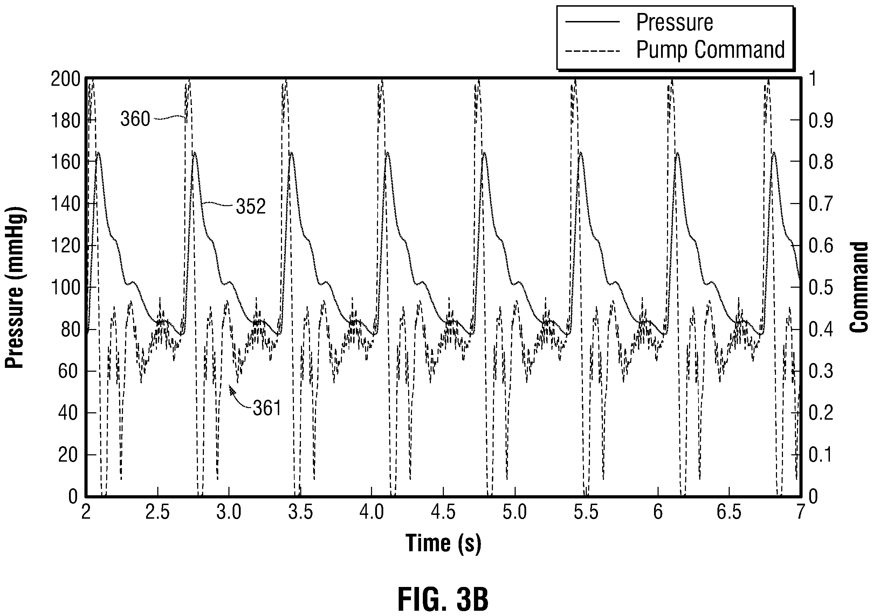

With additional reference to FIG. 3B, another example of the volume clamp mode will be described, in which, a valve controlled by control circuitry 180 to open and close to release pneumatic pressure from the bladder 156 is not utilized, and a fixed orifice 179 including vent 177 not controlled by controlled circuity 180, to passively release pneumatic pressure from the bladder 156 is utilized. In FIG. 3B, the X-axis shows time (seconds) and the Y-axis shows pressure (mmHg) and command values (e.g., 0-1). For example, line 352 shows pressure generated in the bladder 156 of the finger cuff 104 by the pump 172 applying pneumatic pressure under the control of control circuitry 180, as a function of time. Adjacent to the bladder pressure shown by line 352, line 360 shows a pump drive signal commanding when pump 172 is turned on and how hard it is being driven (e.g., 0-1 command value), as commanded by control circuitry 180. It should be noted that pump 172 is turned fully on (e.g., command value 1) when the pressure of the bladder 156 rises steeply and then the pump drive commands fluctuate up and down 361 at lower command levels as the bladder pressure 352 descends. It should be noted that no valve commands are needed as the fixed orifice 179 passively releases the pneumatic pressure from the bladder.

It should be appreciated that various types of pumps 172 may be utilized to implement the previously described embodiments of the invention. For example, in one embodiment, a piezoelectric pump may be utilized as pump 172. A piezoelectric pump 172 may be of relatively small size, low power consumption, and may operate at high operating frequencies well outside of the human auditory range. Therefore, the piezoelectric pump 172 being of small size and low power consumption is suitable for use in the previously described blood pressure measurement controller 120 that is easily mountable on a patient's finger, hand, wrist, etc., as previously described, in very close proximity to the finger cuff 104. Also, since a piezoelectric pump 172 is relatively quiet it does not interfere with a patient's sleep. Additionally, the piezoelectric pump's 172 ability to be modulated (e.g., quickly turned on and off) to control the pneumatic pressure applied to the bladder 156 of the finger cuff 104 may further minimize power usage and maximize control and efficiency.

Other types of pumps 172 that are of small size, low power consumption, and low sound may also be utilized, such as: a voice coil pump, a piston pump, a diaphragm pump, an electrolytic pump, or a peristaltic pump. These types of pumps may be utilized with these same types of advantages as the piezoelectric pump.

In one embodiment, valve 174 may be a piezoelectric valve including a vent 177. Piezoelectric valves, similar to the piezoelectric pump, have the advantages of small size, low power consumption, and high actuation speeds. As one particular example, piezoelectric valve 177 may include a piezoceramic actuator section, a fixed beam section connected to the piezoceramic actuator section, and a rubber sleeve portion connected to the fixed beam section. The piezoceramic actuator section, the fixed beam section, and the rubber sleeve portion may be connected linearly and the rubber sleeve portion may extend over the vent 177. In this way, the valve command signal from the control circuitry 180 may the cause the movement/actuation of the piezoceramic actuator section such that the rubber sleeve portion moves away from the vent 177 (opening) or over the vent 177 (closing) to control the opening and the closing of the vent. As previously described, as an example, a command signal (0-1) may be sent commanding how much the piezoelectric valve should be opened (e.g., how much the piezoceramic actuator section should be commanded to be moved/actuated to simultaneously move the rubber sleeve portion away from the vent to open the vent). It should be appreciated that this is just one example. Other types of valves that have similar characteristics of a piezoelectric valve (e.g., small size, low power consumption, and high actuation speeds), such as, a solenoid valve, an electromechanical valve, etc. It should be appreciated that any type of suitable valve may be utilized.

Various examples will be hereafter described that may be used to implement the previously described blood pressure measurement device that is wearable by a patient.

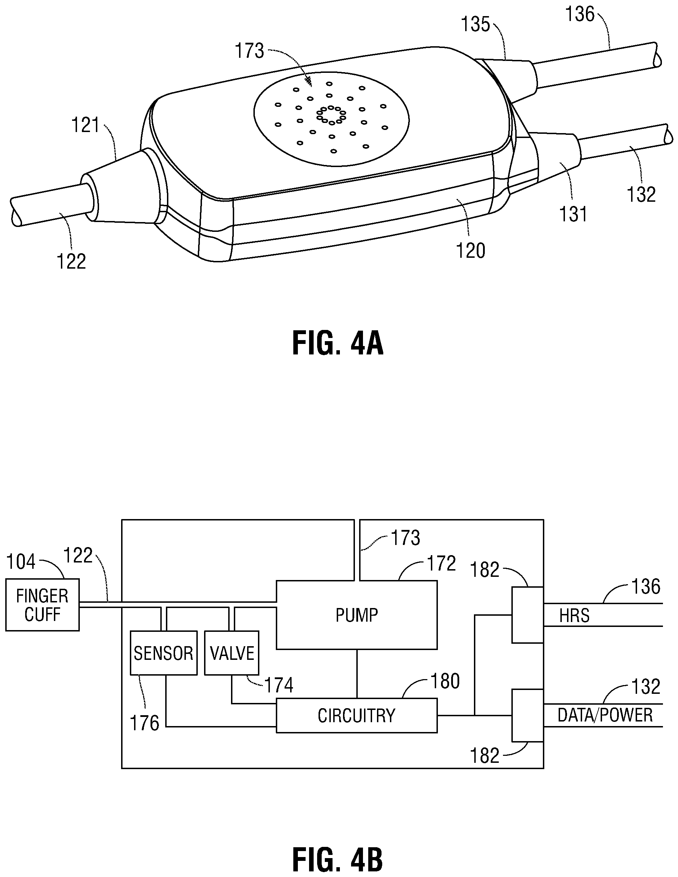

For example, FIG. 4A is a perspective view of the previously described blood pressure measurement controller 120, in which, the finger cuff connector 175 may be a suitable connector 122 including a tube portion to provide the pneumatic pressure from the pump 172 of the blood pressure measurement controller 120 to the bladder 156 of the finger cuff 104 (as well as a cable portion to provide the pleth signal from the finger cuff 104 the control circuity 180). With reference also to FIG. 4B, FIG. 4B illustrates the components of the blood pressure measurement controller 120 contained in the housing of the blood pressure measurement controller 120, as previously described.

As can be seen in FIG. 4A, the blood pressure measurement controller 120 may have an approximately rectangular shaped housing with curved ends and may have air inlets 173 at the top to provide air for pneumatic pressure generated by pump 172. In this example, as has been described, connector 122 may be coupled to the blood pressure measurement controller 120 by a mounting connector 121, in which, connector 122 may include an appropriate tube to provide the pneumatic pressure from the pump 172 of the blood pressure measurement controller 120 to the bladder 156 of the finger cuff 104. Further, blood pressure measurement controller 120 may be coupled to the patient monitoring device 130 through a power/data cable 132 connected by a mounting connector 131 and to the HRS 134 through a HRS connector 136 connected by a mounting connector 135. The patient monitoring device 130 may be any type of medical electronic device that may read, collect, process, display, etc., physiological readings/data of a patient including blood pressure, as well as any other suitable physiological patient readings.

In this example, in which, the finger cuff connector 175 may be an appropriate connector 122 to provide the pneumatic pressure from the pump 172 of the blood pressure measurement controller 120 to the bladder 156 of the finger cuff 104, the operation of the blood controller pressure measurement controller 120 proceeds, as previously described. In particular, control circuitry 180 may be configured to: control the pneumatic pressure applied by the pump 172 (e.g., from air inlet 173) through the connector 122 to the bladder 156 to replicate the patient's blood pressure based upon measuring the pleth signal received from the LED-PD pair of the finger cuff 104. Further, control circuitry 180 may be configured to: control the opening of the valve 174 to release pneumatic pressure from the bladder 156. However, in some example of the disclosure, as previously described, a valve controlled by control circuitry 180 to open and close to release pneumatic pressure from the bladder 156 is not utilized, and a fixed orifice not controlled by controlled circuity 180, to passively release pneumatic pressure from the bladder 156 may be utilized. Additionally, control circuitry 180 may be configured to: measure the patient's blood pressure by monitoring the pressure of the bladder 156 based upon the input from the pressure senor 176, which should be the same as patient's blood pressure. The patient's measured blood pressure may be commanded by the control circuitry 180 to be transmitted through the I/O circuitry and interface 182 through data/power cable 132 to the patient monitoring device 130 to display the patient's measured blood pressure. Further, control circuitry 180 based upon input from HRS 134 and other sources received through the I/O circuitry and interface 182 through HRS connector 136 may allow for compensation of potential errors due to differences in height between the finger cuff 104 and the heart level in the calculation of blood pressure measurements.

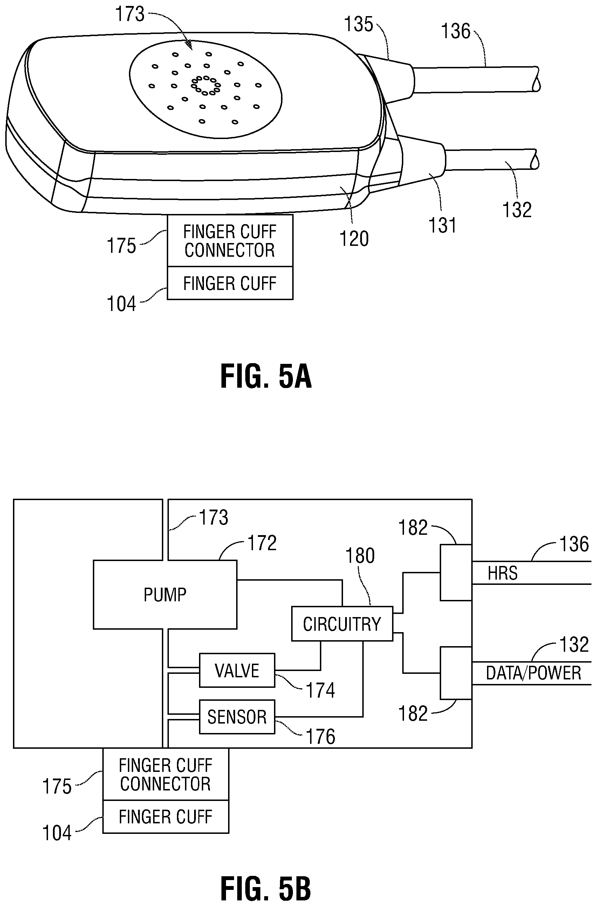

As another example, with reference to FIG. 5A, FIG. 5A is a perspective view of the previously described blood pressure measurement controller 120, in which, the finger cuff connector 175 may be a relatively short connection fixture for directly connecting the pneumatic pressure from the pump 172 of blood pressure measurement controller 120 to the bladder 156 of the finger cuff 104. In this example, the blood pressure measurement controller 120 may be connected directly on top of the finger cuff 104 with the connection fixture such that the whole system (both the blood pressure measurement controller 120 and the figure cuff 104) are placed directly on top of a patient's finger. It should be appreciated that the connection fixture may be any suitable form of mechanical fixture structure (e.g., metallic, plastic, etc.) that is relatively small and that has sufficient strength to securely connect the housing of the blood pressure measurement controller 120 to the housing of the finger cuff 104 and that may include a relatively small interior tube portion to provide the outputted pneumatic pressure from the pump 172 of the blood pressure measurement controller 120 to the bladder 156 of the finger cuff 104 and to support a cable portion to provide the pleth signal from the finger cuff 104 to the control circuity 180. With reference also to FIG. 5B, FIG. 5B illustrates the components of the blood pressure measurement controller 120 contained in the housing of the blood pressure measurement controller 120, as previously described.

As can be seen in FIG. 5A, the blood pressure measurement controller 120 may have an approximately rectangular shaped housing with curved ends and may have air inlets 173 at the top to provide air for pneumatic pressure generated by pump 172. In this example, as has been described, finger cuff connector 175 may be a connection fixture for directly connecting the pneumatic pressure from the pump 172 of blood pressure measurement controller 120 to the bladder 156 of the finger cuff 104. Further, blood pressure measurement controller 120 may be coupled to the patient monitoring device 130 through a power/data cable 132 connected by a mounting connector 131 and to the HRS 134 through a HRS connector 136 connected by a mounting connector 135. The patient monitoring device 130 may be any type of medical electronic device that may read, collect, process, display, etc., physiological readings/data of a patient including blood pressure, as well as any other suitable physiological patient readings.

In this example, in which, the finger cuff connector 175 may be a connection fixture to provide the pneumatic pressure from the pump 172 of the blood pressure measurement controller 120 to the bladder 156 of the finger cuff 104, the operation of the blood pressure measurement controller 120 proceeds, as previously described. In particular, control circuitry 180 may be configured to: control the pneumatic pressure applied by the pump 172 (e.g., from air inlet 173) through the connection fixture 175 to the bladder 156 to replicate the patient's blood pressure based upon measuring the pleth signal received from the LED-PD pair of the finger cuff 104. Further, control circuitry 180 may be configured to: control the opening of the valve 174 to release pneumatic pressure from the bladder 156. However, in some example of the disclosure, as previously described, a valve controlled by control circuitry 180 to open and close to release pneumatic pressure from the bladder 156 is not utilized, and a fixed orifice not controlled by controlled circuity 180, to passively release pneumatic pressure from the bladder 156 may be utilized. Additionally, control circuitry 180 may be configured to: measure the patient's blood pressure by monitoring the pressure of the bladder 156 based upon the input from the pressure senor 176, which should be the same as patient's blood pressure. The patient's measured blood pressure may be commanded by the control circuitry 180 to be transmitted through the I/O circuitry and interface 182 through data/power cable 132 to the patient monitoring device 130 to display the patient's measured blood pressure. Further, control circuitry 180 based upon input from HRS 134 and other sources received through the I/O circuitry and interface 182 through HRS connector 136 may allow for compensation of potential errors due to differences in height between the finger cuff 104 and the heart level in the calculation of blood pressure measurements.

It should be appreciated that various other alternative implementations may be utilized. For example, in one alternative implementation, the valve 174 may simply be a fixed orifice to release pneumatic pressure from the bladder 156. In this implementation, the opening and closing of the valve 174 will therefore not be controlled by the control circuitry 180, as previously described, as it is simply an orifice. In this instance, pneumatic pressure applied to the bladder 156 will be completely controlled and modulated by the pump 172 as controlled by the control circuitry 180. In another implementation, backward leakage of pneumatic pressure out of the pump 172 may be allowed when the pump 172 is powered off to allow for the release of pneumatic pressure from the bladder 156.

Also, although a wired data cable 132 has been illustrated to transmit the measured patient's blood pressure through a wired connection to the patient monitoring device 130, it should be appreciated that a wireless connection may be utilized to transmit data to and from the patient monitoring device 130 instead of a wired connection.

As previously described, blood pressure measurement device 102 utilizing a finger cuff 104 in conjunction with a relatively small and compact blood pressure measurement controller 120 that includes: an internal controllable valve 174, an internal controllable pump 172, and a sensor 176; enables a very compact pressure drive system and feedback loop for a continuous non-invasive blood pressure monitoring system. Further, this type of blood pressure measurement device 102 may simply be located on the patient's wrist, hand, or finger, or may be located in close proximity thereto (e.g., the blood pressure measurement controller 120 may be mounted to a nearby fixture near the finger cuff 104 or may dangle from the finger cuff 104).

Moreover, the compact size of the internal controllable pump 172 (e.g., a piezoelectric pump) and the internal controllable valve 174 (e.g., a piezoelectric valve) being utilized in the blood pressure measurement controller 120 closely coupled to the finger cuff 104 such that the blood pressure measurement device 102 is mountable to patient's finger, hand, wrist, etc., enables a continuous blood pressure measurement system that is portable and can be worn continuously by a patient as the patient moves through the hospital (e.g., from operating room (OR) to intensive care unit (ICU)), or from an ambulance to the emergency room (ER), ICU, etc., or between any locations in a hospital, medical, or home environment, etc. Therefore, by utilizing an internal controllable pump 172 (e.g., a piezoelectric pump) and an internal controllable valve 174 (e.g., a piezoelectric valve) in the blood pressure measurement device 102, a small, compact, and portable blood pressure measurement system is provided. This type of more compact blood pressure measurement device has various advantages as to current systems, such as: provides a high-fidelity feedback loop; is less obtrusive in the OR, ER, ICU, etc.; is of lower cost; is very portable; utilizes less energy; creates less sound - is quieter; etc. In particular, in one implementation, the small size and lightweight of the previously described blood pressure measurement device allows for the easy movability of the patient around a medical facility, as well as, in and out of the medical facility.

It should be appreciated that aspects of the invention previously described may be implemented in conjunction with the execution of instructions by processors, circuitry, controllers, control circuitry, etc., such as control circuitry 180, I/O circuity 182, etc. As an example, control circuity 180 may operate under the control of a program, algorithm, routine, or the execution of instructions to execute methods or processes in accordance with embodiments of the invention previously described. For example, such a program may be implemented in firmware or software (e.g. stored in memory and/or other locations) and may be implemented by processors, control circuitry, and/or other circuitry, these terms being utilized interchangeably. Further, it should be appreciated that the terms processor, microprocessor, circuitry, control circuitry, circuit board, controller, microcontroller, etc., refer to any type of logic or circuitry capable of executing logic, commands, instructions, software, firmware, functionality, etc., which may be utilized to execute embodiments of the invention.

The various illustrative logical blocks, processors, modules, and circuitry described in connection with the embodiments disclosed herein may be implemented or performed with a general purpose processor, a specialized processor, circuitry, a microcontroller, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A processor may be a microprocessor or any conventional processor, controller, microcontroller, circuitry, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module/firmware executed by a processor, or any combination thereof. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such the processor can read information from, and write information to, the storage medium. In the alternative, the storage medium may be integral to the processor.

The previous description of the disclosed embodiments is provided to enable any person skilled in the art to make or use the present invention. Various modifications to these embodiments will be readily apparent to those skilled in the art, and the generic principles defined herein may be applied to other embodiments without departing from the scope of the appended claims. Thus, the present invention is not intended to be limited to the embodiments shown herein, instead the scope of protection is defined by the appended claims.

## Claims

1. A blood pressure measurement device that is wearable by a patient, comprising:
a finger cuff (104) attachable to the patient's finger including a light emitting diode (LED) (150) - photodiode (PD) (152) pair to measure a pleth signal and a bladder (156) surrounding the finger; and
a blood pressure measurement controller (120) coupled to the bladder (156) through a finger cuff connector (175) to provide pneumatic pressure to the bladder (156), the blood pressure measurement controller (120) wearable by the patient, the blood pressure measurement controller (120) including: a pump (172) coupled to the finger cuff connector (175), a valve (174), a pressure sensor (176), and control circuitry (180) coupled to the pump (172), the valve (174), the pressure sensor (176), and the LED-PD (150, 152) pair, the control circuitry (180) being configured to:
control the pneumatic pressure applied by the pump (172) through the finger cuff connector (175) to the bladder (156) to replicate the patient's blood pressure based upon measuring the pleth signal;
control the opening of the valve (174) to release pneumatic pressure from the bladder (156); and
measure the patient's blood pressure by monitoring the pressure of the bladder (156) with the pressure sensor (176);
wherein the finger cuff connector (175) includes a connection fixture for directly connecting the pneumatic pressure from the blood pressure measurement controller (120) to the bladder (156) of the finger cuff (104) and wherein the blood pressure measurement controller (120) is mountable on top of the finger cuff (104).

2. The blood pressure measurement device of claim 1, wherein the pump (172) comprises a piezoelectric pump, preferably wherein the piezoelectric pump output is modulated to control pneumatic pressure and minimize power usage, or
wherein the pump (172) comprises at least one of a voice coil pump, a piston pump, a diaphragm pump, an electrolytic pump, or a peristaltic pump.

3. The blood pressure measurement device of claim 1, wherein the valve (174) comprises at least one of a piezoelectric valve, a solenoid valve or an electromechanical valve.

4. The blood pressure measurement device of claim 1, wherein the valve (174) comprises a fixed orifice to release pneumatic pressure in the bladder (156) and wherein pneumatic pressure is modulated by the pump (172).

5. The blood pressure measurement device of claim 1, further comprising allowing backward leakage of pneumatic pressure out of the pump (172) when the pump (172) is powered off to release pneumatic pressure in the bladder (156).

6. The blood pressure measurement device of claim 1, wherein the control circuitry (180) further comprises a data interface (182) to transmit the measured patient's blood pressure through a wired or wireless connection.

7. A method for blood pressure measurement utilizing a finger cuff (104) of a blood pressure measurement device as defined in any of claims 1 to 6, the finger cuff (104) being attachable to a patient's finger and including a light emitting diode (LED) (150) - photodiode (PD) (152) pair to measure a pleth signal and a bladder (156) surrounding the finger, the method comprising:
attaching a blood pressure measurement controller (120) of the blood pressure measurement device to the patient by mounting the blood pressure measurement controller (120) on top of the finger cuff (104), the blood pressure measurement controller (120) directly coupled to the bladder (156) of the finger cuff (104) through a connection fixture of a finger cuff connector (175) to provide pneumatic pressure to the bladder (156), the blood pressure measurement controller (120) including: a pump (172) coupled to the finger cuff connector (175), a valve (174), and a pressure sensor (176);
controlling the pneumatic pressure applied by the pump (172) through the finger cuff connector (175) to the bladder (156) to replicate the patient's blood pressure based upon measuring the pleth signal;
controlling the opening of the valve (174) to release pneumatic pressure from the bladder (156); and
measuring the patient's blood pressure by monitoring the pressure of the bladder (156) with the pressure sensor (176).

## Patentansprüche

1. Blutdruckmessvorrichtung, die von einem Patienten getragen werden kann, umfassend:
eine Fingermanschette (104), die am Finger des Patienten angebracht werden kann und ein Paar aus einer Leuchtdiode (LED) (150) und einer Photodiode (PD) (152) zur Messung eines Pleth-Signals, sowie einen den Finger umgebenden Luftsack (156) enthält; und
eine Blutdruckmess-Steuereinrichtung (120), die über einen Fingermanschettenverbinder (175) mit dem Luftsack (156) gekoppelt ist, um den Luftsack (156) mit pneumatischem Druck zu versorgen, wobei die Blutdruckmess-Steuereinrichtung (120) von dem Patienten getragen werden kann und die Blutdruckmess-Steuereinrichtung (120) umfasst: eine Pumpe (172), die mit dem Fingerdruckmanschettenverbinder (175) gekoppelt ist, ein Ventil (174), einen Drucksensor (176) und eine Steuerschaltung (180), die mit der Pumpe (172), dem Ventil (174), dem Drucksensor (176) und dem LED-PD-Par (150, 152) gekoppelt ist, wobei die Steuerschaltung (180) dazu konfiguriert ist:
den von der Pumpe (172) über den Fingermanschettenverbinder (175) auf den Luftsack (156) ausgeübten pneumatischen Druck zu steuern, um den Blutdruck des Patienten basierend auf der Messung des Pleth-Signals zu replizieren;
die Öffnung des Ventils (174) zu steuern, um den pneumatischen Druck aus dem Luftsack (156) abzulassen; und
den Blutdruck des Patienten zu messen, indem der Druck des Luftsacks (156) mit dem Drucksensor (176) überwacht wird;
wobei der Fingermanschettenverbinder (175) eine Verbindungsbefestigung zum direkten Verbinden des pneumatischen Drucks von der Blutdruckmess-Steuereinrichtung (120) mit dem Luftsack (156) der Fingermanschette (104) aufweist, und wobei die Blutdruckmess-Steuereinrichtung (120) auf der Oberseite der Fingermanschette (104) montierbar ist.

2. Blutdruckmessvorrichtung nach Anspruch 1, wobei die Pumpe (172) eine piezoelektrische Pumpe umfasst, wobei vorzugsweise die Leistung der piezoelektrischen Pumpe moduliert wird, um den pneumatischen Druck zu steuern und den Energieverbrauch zu minimieren, oder
wobei die Pumpe (172) wenigstens eine umfasst von einer Schwingspulenpumpe, einer Kolbenpumpe, einer Membranpumpe, einer elektrolytischen Pumpe oder einer peristaltischen Pumpe.

3. Blutdruckmessvorrichtung nach Anspruch 1, wobei das Ventil (174) wenigstens eines umfasst von einem piezoelektrischen Ventil, einem Solenoidventil oder einem elektromechanischen Ventil.

4. Blutdruckmessvorrichtung nach Anspruch 1, wobei das Ventil (174) eine feste Öffnung umfasst, um den pneumatischen Druck im Luftsack (156) abzulassen, und wobei der pneumatische Druck durch die Pumpe (172) moduliert wird.

5. Blutdruckmessvorrichtung nach Anspruch 1, die ferner das Ermöglichen des Entweichens des pneumatischen Drucks aus der Pumpe (172) nach hinten umfasst, wenn die Pumpe (172) ausgeschaltet ist, um den pneumatischen Druck im Luftsack (156) abzulassen.

6. Blutdruckmessvorrichtung nach Anspruch 1, wobei die Steuerschaltung (180) ferner eine Datenschnittstelle (182) umfasst, um den gemessenen Blutdruck des Patienten über eine verdrahtete oder drahtlose Verbindung zu übermitteln.

7. Verfahren zur Blutdruckmessung unter Verwendung einer Fingermanschette (104) einer Blutdruckmessvorrichtung nach einem der Ansprüche 1 bis 6, wobei die Fingermanschette (104) am Finger eines Patienten angebracht werden kann und ein Paar aus einer Leuchtdiode (LED) (150) und einer Photodiode (PD) (152) zur Messung eines Pleth-Signals, sowie einen den Finger umgebenden Luftsack (156) aufweist, wobei das Verfahren umfasst:
Anbringen einer Blutdruckmess-Steuereinrichtung (120) der Blutdruckmessvorrichtung an dem Patienten durch Montieren der Blutdruckmess-Steuereinrichtung (120) oben auf der Fingermanschette (104), wobei die Blutdruckmess-Steuereinrichtung (120) direkt mit dem Luftsack (156) der Fingermanschette (104) über eine Verbindungsbefestigung eines Fingermanschettenverbinders (175) gekoppelt ist, um den Luftsack (156) mit pneumatischem Druck zu versorgen, wobei die Blutdruckmess-Steuereinrichtung (120) umfasst: eine Pumpe (172), die mit dem Fingermanschettenverbinder (175) gekoppelt ist, ein Ventil (174) und einen Drucksensor (176);
Steuern des pneumatischen Drucks, der von der Pumpe (172) über den Fingermanschettenverbinder (175) an den Luftsack (156) ausgeübt wird, um den Blutdruck des Patienten basierend auf der Messung des Pleth-Signals zu replizieren;
Steuern der Öffnung des Ventils (174) zum Ablassen des pneumatischen Drucks aus dem Luftsack (156); und
Messen des Blutdrucks des Patienten durch Überwachung des Drucks des Luftsacks (156) mit dem Drucksensor (176).

## Revendications

1. Dispositif de mesure de tension artérielle qui peut être porté par un patient, comprenant :
un doigtier (104) pouvant être attaché au doigt du patient comprenant une paire diode électroluminescente (DEL) (150) - photodiode (PD) (152) destinée à mesurer un signal photopléthysmographique et une poche (156) entourant le doigt ; et
un dispositif de commande (120) de mesure de tension artérielle accouplé à la poche (156) par l'intermédiaire d'un raccord (175) de doigtier destiné à fournir une pression pneumatique à la poche (156), le dispositif de commande (120) de mesure de tension artérielle pouvant être porté par le patient, le dispositif de commande (120) de mesure de tension artérielle comprenant : une pompe (172) accouplée au raccord (175) de doigtier, une vanne (174), un capteur de pression (176) et un circuit de commande (180) accouplé à la pompe (172), à la vanne (174), au capteur de pression (176) et à la paire de DEL-PD (150, 152), le circuit de commande (180) étant conçu pour :
commander la pression pneumatique appliquée par la pompe (172) par l'intermédiaire du raccord (175) de doigtier à la poche (156) afin de reproduire la tension artérielle du patient sur la base de la mesure du signal photopléthysmographique ;
commander l'ouverture de la vanne (174) afin de libérer la pression pneumatique de la poche (156) ; et
mesurer la tension artérielle du patient tout en surveillant la pression de la poche (156) à l'aide du capteur de pression (176) ;
le raccord (175) de doigtier comprenant un appareil de raccordement destiné à raccorder directement la pression pneumatique à partir du dispositif de commande (120) de mesure de tension artérielle à la poche (156) du doigtier (104) et le dispositif de commande (120) de mesure de tension artérielle pouvant être monté sur le dessus du doigtier (104).

2. Dispositif de mesure de tension artérielle selon la revendication 1, la pompe (172) comprenant une pompe piézoélectrique, de préférence la sortie de la pompe piézoélectrique étant modulée afin de commander la pression pneumatique et de réduire au minimum l'utilisation d'énergie ou
la pompe (172) comprenant au moins une pompe parmi une pompe à bobine acoustique, une pompe à piston, une pompe à membrane, une pompe électrolytique ou une pompe péristaltique.

3. Dispositif de mesure de tension artérielle selon la revendication 1, la vanne (174) comprenant au moins une vanne parmi une vanne piézoélectrique, une électrovanne ou une vanne électromécanique.

4. Dispositif de mesure de tension artérielle selon la revendication 1, la vanne (174) comprenant un orifice fixe destiné à libérer la pression pneumatique dans la poche (156) et la pression pneumatique étant modulée par la pompe (172).

5. Dispositif de mesure de tension artérielle selon la revendication 1, comprenant en outre le fait de permettre une fuite vers l'arrière de la pression pneumatique hors de la pompe (172) lorsque la pompe (172) est mise hors tension afin de libérer la pression pneumatique dans la poche (156).

6. Dispositif de mesure de tension artérielle selon la revendication 1, le circuit de commande (180) comprenant en outre une interface de données (182) destinée à transmettre la tension artérielle mesurée du patient par l'intermédiaire d'une connexion filaire ou sans fil.

7. Procédé de mesure de tension artérielle à l'aide d'un doigtier (104) d'un dispositif de mesure de tension artérielle tel que défini dans l'une quelconque des revendications 1 à 6, le doigtier (104) pouvant être attaché au doigt d'un patient et comprenant une paire diode électroluminescente (DEL) (150) - photodiode (PD) (152) destinée à mesurer un signal photopléthysmographique et une poche (156) entourant le doigt, le procédé comprenant :
l'attache d'un dispositif de commande (120) de mesure de tension artérielle du dispositif de mesure de tension artérielle au patient par montage du dispositif de commande (120) de mesure de tension artérielle sur le dessus du doigtier (104), le dispositif de commande (120) de mesure de tension artérielle étant directement accouplé à la poche (156) du doigtier (104) par l'intermédiaire d'un appareil de raccordement d'un raccord (175) de doigtier afin de fournir une pression pneumatique à la poche (156), le dispositif de commande (120) de mesure de tension artérielle comprenant : une pompe (172) accouplée au raccord (175) de doigtier, une vanne (174) et un capteur de pression (176) ;
la commande de la pression pneumatique appliquée par la pompe (172) par l'intermédiaire du raccord (175) de doigtier à la poche (156) afin de reproduire la tension artérielle du patient sur la base de la mesure du signal photopléthysmographique ;
la commande de l'ouverture de la vanne (174) afin de libérer la pression pneumatique de la poche (156) ; et
la mesure de la tension artérielle du patient par surveillance de la pression de la poche (156) à l'aide du capteur de pression (176).
